# EUROPEAN PATENT SPECIFICATION

(11) **EP 1 799 296 B1**
(45) Date of publication and mention of the grant of the patent: **31.03.2010**
(21) Application number: 05787190.7
(22) Date of filing: 14.09.2005
(51) Int. Cl.: A61N 1/05

(54) **IMPLANTABLE FIXATION ELEMENT**
IMPLANTIERBARES FIXIERELEMENT
ELEMENT DE FIXATION IMPLANTABLE

(30) Priority: 28.09.2004 NL 1027131
(43) Date of publication of application: 27.06.2007
(73) Proprietor: Cardiall B.V., CR Varsseveld (NL)
(72) Inventor: VAN DER KEMP, Peter, NL-3218 LB Heenvliet (NL); VERHEUL, Simon, Alexander, NL-6511 TJ Nijmegen (NL); GEENEN, Kamiel,Reinier, Zale, NL-6511 TJ Nijmegen (NL)
(74) Representative: Griebling, Onno
(86) International application number: PCT/NL2005/000670
(87) International publication number: WO 2006/036058

(56) References cited:
- US-A- 4 142 530
- US-A- 4 144 890
- US-A- 5 242 457
- US-A1- 2003 220 678
- US-A1- 2004 176 830

## Description

The invention relates to an implantable fixation element for anchoring at least one elongate,for instance essentially cylindrical element in human or animal tissue, comprising a pair of first gripper beaks, movable to and from each other so as to enclose this element, and a fixing part, interconnected therewith and destined for engaging the tissue.

Such a fixation element is known from the published US Patent Application US2003/0,220,678 A1. This known fixation element, which is made from spring steel, comprises two beaks of which the respective ends cross each other and merge into the legs of a hairpin spring, so that the beaks are biased with a certain force towards each other.

This fixation element is used for anchoring flexible elongated and insulated electrical leads to the wall of a stomach; the free ends of the leads come to lie against the wall of the stomach and are connected to a suitable source of electrical stimulating pulses. To maintain the correct position of the ends a first fixation element is placed around the pair of leads and in the stomach, lying against the inner wall of the stomach while a second, similar fixation element, also lying against the outer wall of the stomach, is placed around the pair of leads but outside the stomach. In this way the electrodes are anchored there where the enter the stomach.

The problem of fixing insulated leads which carry electrical stimulating pulses to the surrounding tissue also occurs in the technology of pacemakers ,implantable defibrillators and implantable cardioverters. There a supple, electrical lead is inserted by means of a catheter and through a vein, in most cases the vein which lies beneath the collar bone, and is then inserted into the hart for supplying the stimulating pulses. This electrical lead must be anchored to the surrounding tissue in de vicinity of the place where the lead exits from the vein. Although in the past, when pacemakers were firstly introduced, the surgeon used to fix the lead very simply by means of surgical stitching wire to the breast muscle, involving many risks, particularly the risk of destroying the insulation of the lead (which has, of course, disastrous results for the correct operation of a pacemaker ,a defibrillator or a cardioverter), presently this anchoring is commonly done using a special protecting sleeve around the lead which is provided with circumference grooves, and this protecting sleeve is then fixed to the surrouding tissue by the surgeon by means of surgical stitching wire. This technique is described in PACE, vol. 15, November 1992, page 2005-2010, PACE, vol. 21, March 1998, page 549-552 and in the publication "A practical approach to permanent pacemaker implantation", Peter H. Belott, M.D., Futura Publishing Company, Armonck, NY, USA, 1995 (ISBN 087973-594-4).

This procedure is, however, not an ideal one and obtaining a correct fixation is very much dependent upon the skill of the surgeon. Even when one uses the abovementioned protecting sleeve the final fixation is done by means of stitching wire and surgical knots, thus by manipulation, during which errors can be made, particularly by an incorrect tensioning of the wire (too tight or too loose), or by using incorrect knots.

Theoretically it could be possible to use the fixation element as known from the abovementioned US Patent Application US2003/0,220,678 A1 to anchor the electrical leads as used with a cardial pacemaker to the surrounding tissue but in that case, too, the surgeon must anchor the fixation device by means of stitching wire to the surrounding tissue - with all the drawbacks which this implies -, while, to obtain a correct fixation, the beaks of the fixation device must exercise a quite strong force on the lead which they enclose. As a result of the fact that the fixation device must be made from spring steel to obtain a sufficient pressure there is a very great risk of damage to the insulation of the leads, so that the metal can contact the electrically conducting core thereof, with damaging and, possibly fatal results.

The invention aims to obviate the above described drawbacks and particularly to provide a fixation element to be made from plastics which in a simple way and with one single operation encloses both the cylindrical element and anchors this to the surrounding tissue. This object is achieved according to the invention in that this fixating part comprises at least a pair of second fixation gripper beaks, capable for movement together with the first pair around a common point of rotation for a simultaneous opening and closing thereof and having an open configuration with opposite ends, of which second gripper beaks the ends, during closing, and capable to penetrate the tissue while means are provided to lock the beaks with respect to each other in the closed state.

As a result of the forced and simultaneous movement to each other of both the gripper beaks intended to surround the element to be anchored as well as the fixation grippers which enclose with their sharp ends the tissue it is ensured that in one step the element is enclosed reliably while at the same time this element is reliably anchored to the surrounding tissue.

An advantageous embodiment is obtained when the first and second pair of gripper beaks lie at the same side of a common point of rotation.

An embodiment which can be easily fabricated in an advantageous way is one in which the respective gripper beaks of the first and the second pair respectively are each formed by a first and second respectively hinge arm, hingedly interconnected at one end, each such hinge arm having, starting from the hinge point, a first part-circular cut-out, said first cut-outs defining together the pair of first gripper beaks, with adjoining a second part-circular cut-out, said second cut-outs defining together the second pair of fixation gripper beaks, while, between the first and second cut-outs respectively, interacting catching coupling parts are provided.

Preferably the two hinge arms are made in one piece and each hinge point is made up by a thin strip of flexible material.

Preferably each hinge arm is at its outer side provided with a out-cut to cooperate with a applicator excerting an inwardly directed force thereon.

A fixation element can be connected detachably to a part of this applicator, such as described in claim 6, resulting into a disposable unit which can be sterilized as such.

It is observed that staples for surgical uses, of which the legs have, in cross-section, a C-shaped configuration and of which the points can penetrate the tissue when a force is excerted on the legs are known in itself, for instance from the US patent specifications 5,242,457, upon which prior art the two part form of claim 1 is based, and 5,618,311, the US patent application 2003/0,111,641 A1 and the European patent application 0 565 892 A1. Applicators for surgical staples are also known - vide for instance the US patent specification 6,059,799 and the International patent application WO99/18857.

US 4 144 890 discloses a contact device for muscle stimulation,particularly an epicardial electrode,having a pair of spaced-apart prong-like attachment members,and also discloses an applicator tool for positioning same.The use of prong-like attachment members on an epicardial electrode is furthermore disclosed in US 2004/0 176 830 A1,as well as in US 4 142 530 which describes an epicardial lead with an electrode tip provided with such members.

However, none of these publications discloses the subject of the present application, while this subject also cannot be considered to be obvious after having taken notice of these publications.

The invention is elucidated on the hand of the drawing.
Fig. 1 shows on a very enlarged scale a front view of a fixation element according to the invention in the open state.
Fig. 2 shows a front view of this element, now in the locked, and closed state.
Fig. 3 shows a front view corresponding to the view according to fig. 1, but now of the fixation element combined with a, protecting sleeve preferably to be used therewith, and with a cylindrical element to be fixed therewith.

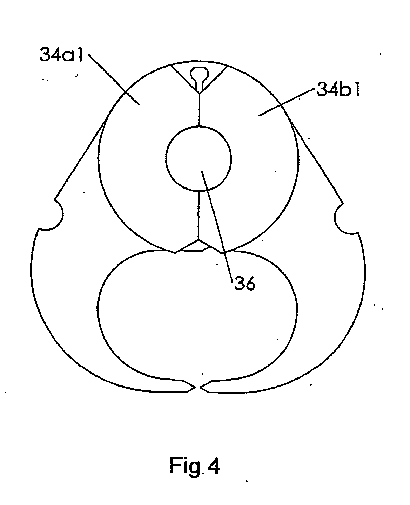

Fig. 4 shows a similar front view with the fixation element in the closed state.
Fig. 5 is a perspective view of the combination: fixation element, protecting sleeve and element to be enclosed thereby.
Fig. 6-10 are front views of the principle parts of an applicator to be used in combination with the fixation element in the various operative states thereof.
Fig. 11 shows a side view of an applicator with, shown there beneath, an element fixed to tissue.

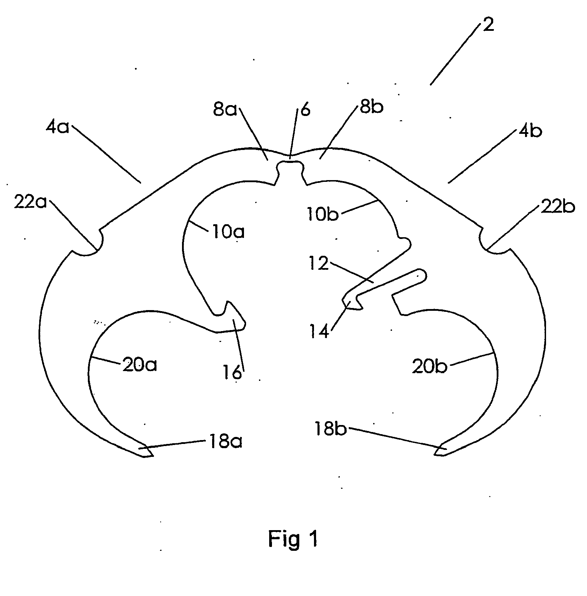

In fig. 1 the fixation element is indicated in its entirety by reference numeral 2; it is made in one piece, for instance by molding, from a suitable plastics and comprises a first arm 4a and a second arm 4b, of which the respective ends 8a, 8b are interconnected hingedly by means of a thin strip of material 6. Both the arms have a first partly circular cut-out 10a, 10b, lying close to the interconnecting point 6, with directly there under a locking mechanism comprising a biased hooking arm 12, protruding from the arm 4b, with the cross-hook 14, cooperating with and a take-up hook 16, protruding from the arm 4a. The arms 4a, 4b end in pointed teeth 18a, 18b and there is, between the tooth 18a and the take-up hook 16 as well as between the tooth 18b and the locking hook 12 a second partly circular cut-out, indicated with 20a and 20b, respectively.

Both the arm 4a and the arm 4b has at the outer circumference a cut-out 22a and 22b, respectively; the purpose thereof will be described later on.

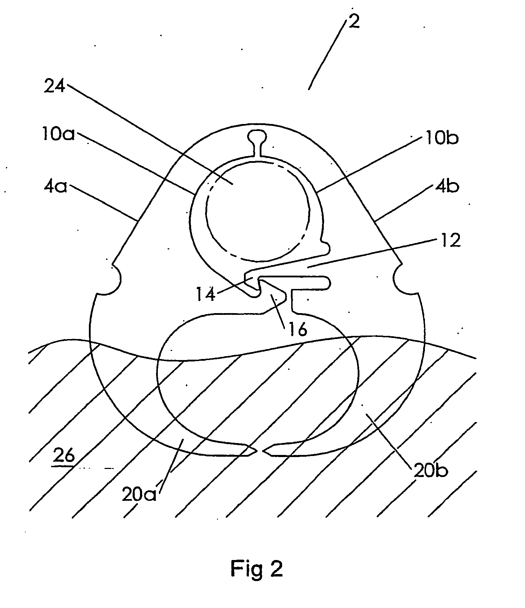

Fig. 2 shows the fixation element in the closed state, in which the arms 4a, 4b are moved towards each other and the hook end 14 hooks around the take-up hook 16. It is clear that the cut-outs 10a, 10b in combination with the hookarm 12 define between them an essentially cylindrical space in which a schematically indicated element 24,here shown as an elongate circelcylindrical element , can be enclosed while, when the pointed teeth 18a, 18b during the closing of the fixation element had rested with some force on the upper surface of a tissue 26, these teeth have by now penetrated this tissue to a certain depth. In this way the fixation element is anchored to the tissue and the cylindrical element 24, too, is anchored to this tissue.

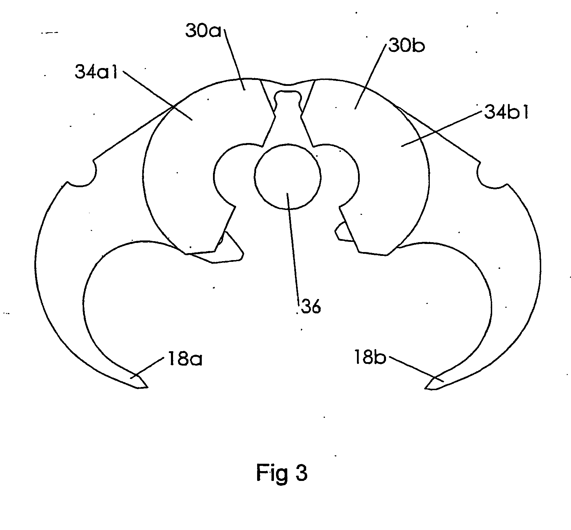

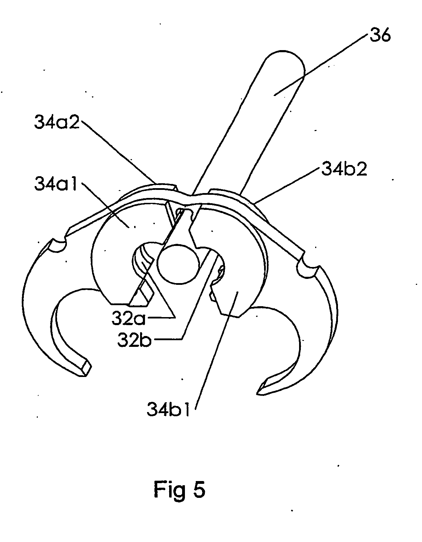

In the medical practice and certainly when the fixation element is used to anchor to tissue electrical leads which carry electrical pulses (said lead thus being the cylindrical element to be anchored) one will preferably position between this lead and the fixation element a protecting sleeve, such as shown in the drawings 3 to and including 5. Figure 3 shows such a sleeve which comprises two halves 30a, 30b and each halve - see in particular figure 5 - comprises a central hub-shaped part 32a, 32b, bounded by to end flanges 34a1, 34a2 and 34b1, 34b2, respectively. The space with a cylindrical cross-section as defined by the hub parts 32a, 32b accommodates the cylindrical element which is to be anchored, thus the flexible insulated lead 36 carrying the electrical pulses. It will be clear from the preceding that the fixation element will be attached to the tissue in one operation, namely the movement towards each other of the arms 4a and 4b until the locking elements are intercoupled and the entirety is brought into the closed state, in which the element to be anchored is reliably surrounded and anchored to the tissue in the desired position.

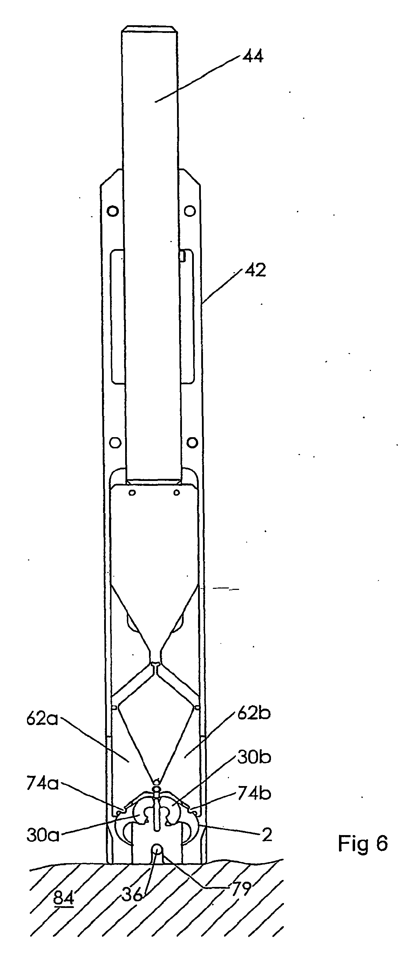

Exercizing the closing forces is preferably done at the position of the cut-outs 22a, 22b in the arms 4a, 4b and a suitable applicator according to the invention by means of which this can be brought about will be elucidated on the hand of figures 6 to and including 11.

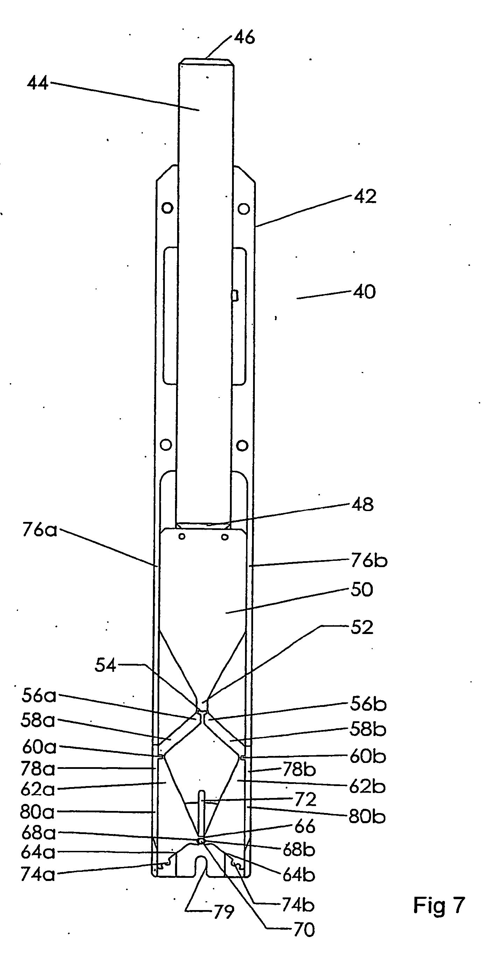

These figures show the parts of the applicator, which is in its entirety indicated with reference numeral 40 - see the figures 6 and 7. These parts are accommodated in an elongated prismatic housing 42 with a greater dimension in the plane of the drawing (the width), then in a direction perpendicular to the plane of the drawing (the thickness). The simple structure of this applicator makes a cheap production thereof possible so that it can be discarded after use.

The housing 42 accommodates slidingly an elongate pressure pin 44 of which during use the upper end 46 must be pressed downwardly and of which the lower end 48 bears on a sliding actuator 50; the lower end 52 thereof is hingedly (at the position 54a, 54b) connected to the upper ends 56a, 56b of two pressure arms 58a, 58b, the narrowing lower ends 60a, 60b thereof merge into two triangular pressure pieces 62a, 62b, each having the shape of an obtuse triangle with long sides and a short basis, 64a, 64b. The obtuse points thereof are, at 66, interconnected and beneath this interconnecting point cut-outs 68a, 68b enclose a guide pin 70. This guide pin 70 is thus perpendicular to the plane of the drawing and is guided in two narrow, guiding slits 72, which lie in the longitudinal axis of symmetry and are provided in the front and rear wall (perpendicular to the plane of the drawing) of the applicator 40. From each basis 64a, 64b protudes a cam indicated with 74a, 74b, respectively, intended for cooperation with a cut-out 22a, 22b of a fixation element which is to be applicated. The front wall 73a and the rear wall 73b of the housing are at the narrowed lower end 77 provided with an entry slit 79, by means of which the applicator is placed around a cylindrical element 76 which is to be put into place while furthermore each of the sidewalls 76a, 76b has an elongated cut-out 78a, 78b, the purpose of which will be described later on.

It is observed that the actuator 40, the arms 58a, 58b, interconnected therewith and the two triangular pressure pieces 62a, 62b can be made up in one piece from a suitable plastics, while the hinges 60a, 60b and 68a, 68b consist of parts with a small thickness of the material, so that there a so-called "bending hinge" is obtained.

Fig. 6 shows the initial position in which the pressure pin 44 protrudes as far as possible from the housing 42, the triangular pressure pieces 62a, 62b have their upper position and the distance between the cams 74a, 74b is at its maximum. In this state an - open - fixation element 2 can be supplied into the housing 42, said fixation element 2 being provided with the two sleeve halves 30a, 30b (see figure 3). In this state the housing 42 is placed with the feeding slit 79 around the cylindrical element 36 which is to be anchored, with the lower end of the housing resting upon the tissue 84 to which the anchoring must take place.

Fig. 7 shows the respective positions of the various parts in the state in which, by pressing the end of the pressure pin 74, through the actuator 50, the upper ends 56a, 56b of the arms 58a, 58b have just arrowed at the upper ends of the cut-outs 78a, 78b and the hinge pin 70 lies at the lower end of the guide 72. For clarity reasons in this drawing the fixation element 2 is not shown.

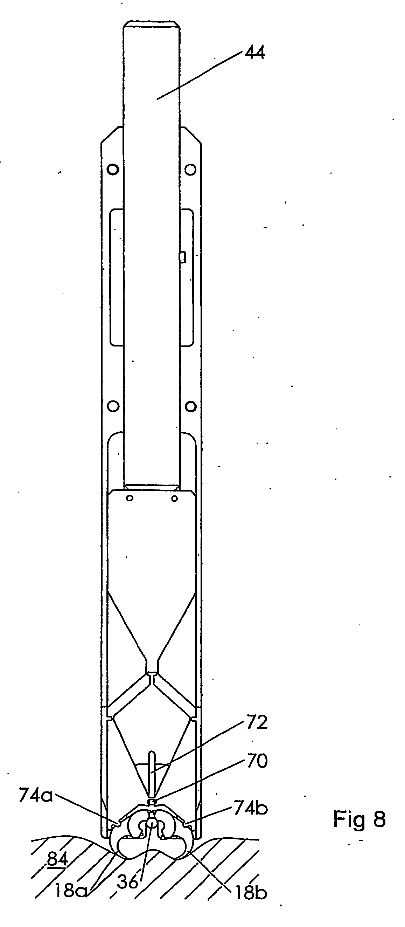

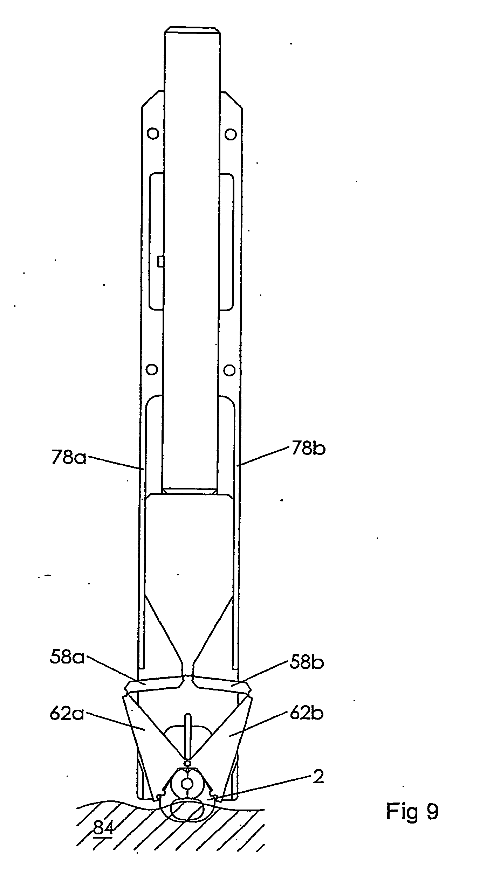

Fig. 8 and 9 show how, by pressing the pin 44, the parts have now reached their lowest position, in which the teeth 18a, 18b rest on the tissue 84. The fixation element however is not yet closed because the mutual distance of the cams 74a, 74b has not changed. The pin 70 has now arrived at the lower end of the guiding slid 72 and as a result the hinge point of the pressure pieces 62a, 62b cannot move further downwardly.

A further downwardly pressing of the pin 77 results into the situation of fig. 9. As the hinge points 60a, 60b between the arm 58a and pressure piece 62a on the one hand, and arm 58b and pressure piece 62b on the other hand now lie there where the cut-outs 78a, 78b in the respective sidewalls 76a, 76b begin, the arms 58a, 58b can move outwardly to the left and right respectively in the plane of the drawing, they move the points of the triangular pressure pieces 62a, 62b with hem in this movement and thus decrease the distance between the cams 74a, 74b. Thus the fixation element 2 is closed while the teeth 18a, 18b thereof penetrate into the underlying tissue.

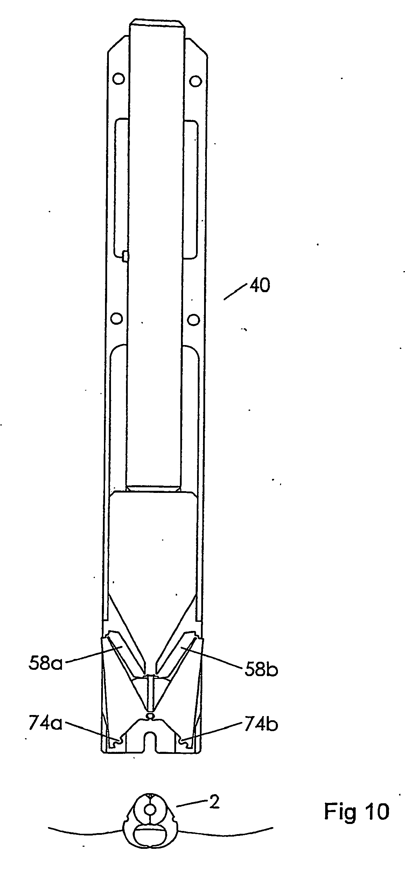

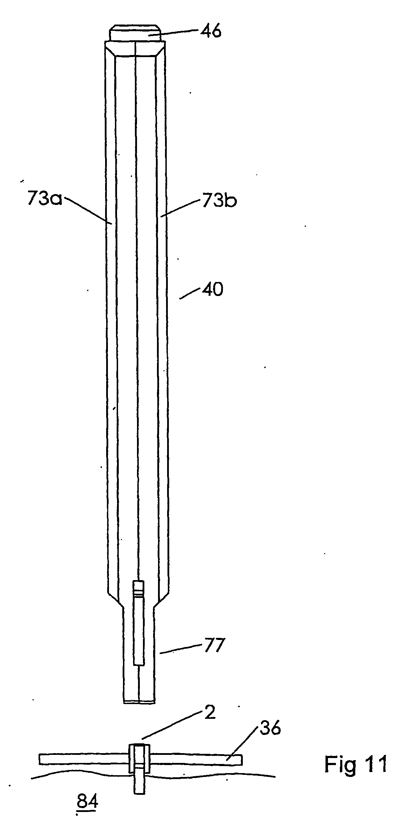

When the pin 44 is pressed downwardly still further the situation according to figure 10 is reached. The arms 58a, 58b are now entirely folded inwardly and the cams 74a, 74b have by now moved away from each other; the applicator 40 can now be taken off, leaving in the tissue 84 the fixation element 2 with the cylindrical element 36 (in particular a pacemaker guide) enclosed thereby such as shown in fig. 10 in front view and in fig. 11 in side view.

It is clear that the application is not limited to the described embodiment thereof; many other uses are possible.

Particularly in the fixation element the shape of the accommodating space can differ from the one which is shown in the drawings, for instance to accommodate an element with a non circular cross-section. Furthermore a fixation element can be interconnected with one of the pressure pieces by means of at least one connection to be ruptured so that it is not necessary to position a loose fixation element in the applicator and as a result one obtains a complete apparatus which is to be used only once. Furthermore two or even more fixation elements can be interconnected and put into place in one operation.

## Claims

1. Implantable fixation element (2) for anchoring at least one elongate, for instance essentially cylindrical, element (24, 36) in human or animal tissue, comprising a pair of first gripper beaks (10a, 10b), movable to and from each other so as to enclose this element, and a fixing part (20a, 20b), interconnected therewith and destined for engaging the tissue, **characterized in that** this fixing part comprises at least a pair of second fixation gripper beaks, capable for movement together with the first pair (10a, 10b) around a common point of rotation (6) for a simultaneous opening and closing thereof and having an open configuration with opposite ends (18a, 18b), of which second gripper beaks the ends, during closing, are capable to penetrate the tissue while means (14, 16) are provided to lock the beaks with respect to each other in the closed state.

2. Fixation element according to claim 1, **characterized in that** the first and second pair of gripper beaks (10a, 10b) lie at the same side of a common point of rotation.

3. Fixation element according to claim 2, **characterized in that** the respective gripper beaks (10a, 10b) of the first and the second pair respectively are each formed by a first and second respectively hinge arm (4a, 4b) hingedly interconnected at one end, each such hinge arm having, starting from the hinge point (6), a first part-circular cut-out (22a, 22b) said first cut-outs defining together the pair of first gripper beaks, with adjoining a second part-circular cut-out, said second cut-outs defining together the second pair of fixation gripper beaks, while, between the first and second cut-outs respectively (22a, 22b), interacting catching coupling parts are provided.

4. Fixation element according to claim 3, **characterized in that** the two hinge arms (4a, 4b) are made in one piece and each hinge point (6) is made up by a thin strip of flexible material.

5. Fixation element according to claim 3-4, **characterized in that** each hinge arm (4a, 4b) is at its outer side provided with a cut-out to cooperate with a applicator exercizing an inwardly directed force thereon.

6. System comprising:
- a fixation element according to any of claims 3-5,
- an applicator to be used in combination with said fixation element, said applicator comprising an elongate housing with a operating element, slidable in the longitudinal direction of the housing and coupled to an actuator, also slidable in the longitudinal direction of the housing, said actuator acting with a central part on the connecting point of the first ends of two pressure arms which include an obtuse angle and of which second ends, which lie in a direction perpendicular to the house axis at a distance of each other can move outwardly through cut-outs in the housing walls and, which each excert a pressure on a tip of an essentially triangular pressure piece of which the respective obtuse basis points are hingedly interconnected and are, movable over a limited distance, guided in the housing in the longitudinal direction thereof, each of said pressure pieces being provided, next to the other basis point for cooperation with a fixation element to be applicated, while the lower end of the housing has a space for accommodating a fixation element to be applicated;
whereby said fixation element is connected with at least one of the pressure pieces of said applicator by means of a rupture connection.

## Patentansprüche

1. Implantierbares Fixierelement (2) zum Verankern zumindest eines länglichen, beispielsweise im Wesentlichen zylindrischen Elements (24, 36) in menschlichem oder tierischem Gewebe, enthaltend ein Paar erster Greifschnabel (10a, 10b), die zueinander und voneinander weg bewegbar sind, um dieses Element zu umschließen, und enthaltend einen Fixierteil (20a, 20b), der damit verbunden und zum Eingriff in das Gewebe bestimmt ist, **dadurch gekennzeichnet, dass** dieser Fixierteil zumindest ein Paar zweiter Fixier-Greifschnabel aufweist, die zur gemeinsamen Bewegung mit dem ersten Paar (10a, 10b) um einen gemeinsamen Drehpunkt (6) in der Lage sind, um sich gleichzeitig zu öffnen und zu schließen, und die eine offene Konfiguration mit gegenüberliegenden Enden (18a, 18b) haben, wobei die Enden der zweiten Greifschnabel während des Schließens in der Lage sind, das Gewebe zu durchdringen, während Mittel (14, 16) vorgesehen sind, um die Schnabel im geschlossenen Zustand in Bezug aufeinander zu verriegeln.

2. Fixierelement nach Anspruch 1, **dadurch gekennzeichnet, dass** das erste und das zweite Paar Greifschnabel (10a, 10b) auf der gleichen Seite eines gemeinsamen Drehpunkts liegen.

3. Fixierelement nach Anspruch 2, **dadurch gekennzeichnet, dass** die entsprechenden Greifschnabel (10a, 10b) des ersten bzw. zweiten Paars jeweils durch erste bzw. zweite Gelenkarme (4a, 4b) gebildet sind, die an einem Ende gelenkig miteinander verbunden sind, wobei jeder solcher Gelenkarm ausgehend vom Gelenkpunkt (6) eine erste, teilweise kreisförmige Aussparung (22a, 22b) aufweist, wobei die ersten Aussparungen gemeinsam das Paar erster Greifschnabel definieren, wobei angrenzend eine zweite, teilweise kreisförmige Aussparung vorgesehen ist, wobei die zweiten Aussparungen gemeinsam das zweite Paar Fixier-Greifschnabel definieren, während zusammenwirkende Rast-Verbindungselemente (22a, 22b) zwischen den ersten bzw. zweiten Aussparungen vorgesehen sind.

4. Fixierelement nach Anspruch 3, **dadurch gekennzeichnet, dass** die beiden Gelenkarme (4a, 4b) einstückig hergestellt sind, und jeder Gelenkpunkt (6) aus einem dünnen Streifen aus flexiblem Material besteht.

5. Fixierelement nach Anspruch 3 und 4, **dadurch gekennzeichnet, dass** jeder Gelenkarm (4a, 4b) an seiner Außenseite mit einer Aussparung versehen ist, um mit einem Applikator zusammenzuwirken, der eine nach innen gerichtete Kraft auf diese ausübt.

6. System, mit:
- einem Fixierelement nach einem der Ansprüche 3 bis 5;
- einen in Kombination mit dem Fixierelement zu verwendenden Applikator, der ein längliches Gehäuse mit einem Bedienelement aufweist, das in Längsrichtung des Gehäuses verschiebbar und mit einem Aktuator verbunden ist, der ebenfalls in Längsrichtung des Gehäuses verschiebbar ist, wobei der Aktuator mit einem Mittelteil auf die Verbindungsstelle der ersten Enden von zwei Druckarmen wirkt, wobei die Druckarme einen stumpfen Winkel aufweisen und sich die zweiten Enden davon, die in einer Richtung normal zur Gehäuseachse in einem Abstand zueinander liegen, durch Aussparungen in den Gehäusewänden nach außen bewegen können, und die jeweils einen Druck auf eine Spitze eines im Wesentlichen dreieckigen Druckstücks ausüben, wobei die jeweiligen stumpfen Basispunkte davon miteinander gelenkmäßig verbunden und über eine begrenzte Distanz bewegbar sind, wobei sie im Gehäuse in Längsrichtung davon geführt werden, wobei jedes Druckstück neben dem anderen Basispunkt zum Zusammenwirken mit einem zu applizierenden Fixierelement vorgesehen ist, während das untere Ende des Gehäuses einen Raum zur Aufnahme eines zu applizierenden Fixierelements aufweist,
wobei das Fixierelement über eine Rupturverbindung mit zumindest einem der Druckstücke des Applikators verbunden ist.

## Revendications

1. Elément de fixation implantable (2) destiné à ancrer au moins un élément allongé (24, 36), par exemple, essentiellement cylindrique, dans des tissus humains ou animaux, et comprenant une paire de premiers becs de serrage (10a, 10b) pouvant se déplacer l'un vers l'autre et en sens opposé l'un à l'autre de manière à entourer cet élément, et une partie de fixation (20a, 20b), reliée mutuellement à ceux-ci et destinée à venir en contact avec les tissus, **caractérisé en ce que** cette partie de fixation comprend au moins une paire de deuxièmes becs de serrage, pouvant se déplacer en même temps que la première paire (10a, 10b) autour d'un point commun de rotation (6) pour une ouverture et une fermeture simultanée de ceux-ci et ayant une configuration ouverte avec des extrémités opposées (18a, 18b) qui sont les extrémités des deuxièmes becs de serrage et qui peuvent, pendant la fermeture, pénétrer dans les tissus alors que des moyens (14, 16) sont prévus pour verrouiller les becs l'un par rapport à l'autre dans l'état fermé.

2. Elément de fixation selon la revendication 1, **caractérisé en ce que** la première et la deuxième paire de becs de serrage (10a, 10b) se trouvent sur le même côté d'un point commun de rotation.

3. Elément de fixation selon la revendication 2, **caractérisé en ce que** les becs de serrage respectifs (10a, 10b) de la première et de la deuxième paire respectivement sont chacun formés par un premier et un deuxième bras d'articulation (4a, 4b), reliés mutuellement de façon articulée au niveau d'une extrémité, chacun de ces bras d'articulation ayant, en partant du point d'articulation (6), une première découpe partiellement circulaire (22a, 22b), lesdites premières découpes définissant ensemble la paire de premiers becs de serrage, avec une deuxième découpe partiellement circulaire, lesdites deuxièmes découpes définissant ensemble la deuxième paire de becs de serrage, alors que, entre les premières et les deuxièmes découpes respectivement (22a, 22b), des parties d'accouplement à accrochage mutuel sont prévues.

4. Elément de fixation selon la revendication 3, **caractérisé en ce que** les deux bras d'articulation (4a, 4b) sont faits d'une seule pièce et chaque point d'articulation (6) est formé par une mince bande de matériau flexible.

5. Elément de fixation selon les revendications 3 à 4, **caractérisé en ce que** chaque bras d'articulation (4a, 4b) est muni, au niveau de son côté extérieur, d'une découpe afin de coopérer avec un applicateur exerçant sur celle-ci une force dirigée vers l'intérieur.

6. Système comprenant :
un élément de fixation selon l'une quelconque des revendications 3 à 5,
un applicateur destiné à être utilisé en association avec ledit élément de fixation, ledit applicateur comprenant un boîtier allongé avec un élément de manoeuvre, pouvant coulisser dans la direction longitudinale du boîtier et couplé à un dispositif d'actionnement, pouvant également coulisser dans la direction longitudinale du boîtier, ledit dispositif d'actionnement agissant avec une partie centrale sur le point de connexion des premières extrémités des deux bras de pression qui comprennent un angle obtus et dont les deuxièmes extrémités, qui se trouvent dans une direction perpendiculaire à l'axe du boîtier à une certaine distance l'une de l'autre, peuvent se déplacer vers l'extérieur par des découpes formées dans les parois du boîtier et, qui exercent chacune une pression sur une extrémité d'une pièce de pression essentiellement triangulaire dont les points de base obtus respectifs sont reliés entre eux de façon articulée et sont, en pouvant se déplacer une sur distance limitée, guidés dans le boîtier dans la direction longitudinale de celui-ci, chacune des pièces de pression étant prévue, près de l'autre point de base pour coopérer avec un élément de fixation devant être mis en place, alors que l'extrémité inférieure du boîtier comporte un espace pour recevoir un élément de fixation devant être mis en place.
